# EUROPEAN PATENT SPECIFICATION

(11) **EP 2 429 950 B1**
(45) Date of publication and mention of the grant of the patent: **17.04.2013**
(21) Application number: 09787591.8
(22) Date of filing: 15.05.2009
(51) Int. Cl.: C01F 17/00, A61K 33/24

(54) **PROCESS FOR THE PREPARATION OF LANTHANUM CARBONATE DIHYDRATE**
VERFAHREN ZUR HERSTELLUNG VON LANTHANCARBONATDIHYDRAT
PROCÉDÉ POUR LA PRÉPARATION DE CARBONATE DE LANTHANE DIHYDRATÉ

(43) Date of publication of application: 21.03.2012
(73) Proprietor: Natco Pharma Limited, Hyderabad 500 033, Andhra Pradesh (IN)
(72) Inventor: MUDDASANI, Pulla Reddy, Hyderabad 500 033 Andhra Pradesh (IN); TALASILA, Sambasiva Rao, Hyderabad 500 033 Andhra Pradesh (IN); ADIBHATLA KALI SATYA, Bhujanga Rao, Hyderabad 500 033 Andhra Pradesh (IN); SATTI, Venkata Reddy, Hyderabad 500 033 Andhra Pradesh (IN); LINGAMANENI, Anil Kumar, Hyderabad 500 033 Andhra Pradesh (IN); NANNAPANENI, Venkaiah Chowdary, Hyderabad 500 033 Andhra Pradesh (IN)
(74) Representative: Srinivasan, Ravi Chandran
(86) International application number: PCT/IN2009/000288
(87) International publication number: WO 2010/131255

(56) References cited:
- EP-A1- 0 622 336
- WO-A2-2007/056721
- US-A- 4 497 785
- US-A- 5 968 976
- LIU, SONG; MA, RONGJUN: "Synthesis and structure characterization of hydrated lanthanum carbonate" HUANAN LIGONG DAXUE XUEBAO - JOURNAL OF SOUTH CHINA UNIVERSITY OF TECHNOLOGY, vol. 25, no. 10, October 1997 (1997-10), pages 23-26, XP8116865

## Description

### FIELD OF INVENTION

Present invention relates to a process for the preparation and pharmaceutical usage of dihydrate form of lanthanum carbonate. Selected Lanthanum carbonate hydrates are used to treat hyperphosphataemia in patients with renal failure. They are administered into the gastrointestinal tract. Shire Pharmaceuticals, under exclusive license from AnorMED, has developed and launched lanthanum carbonate (Fosrenol, formerly Foznol), a phosphate-binding lanthanum salt, for the oral treatment of hyperphosphataemia in dialysis patients. Lanthanum carbonate dihydrate has the formula given below.

La₂(CO₃)₃.xH₂O

Wherein x = 2.0±0.2

### BACKGROUND OF INVENTION

Selected Lanthanum carbonate hydrates of formula La₂(CO₃)₃.xH₂O where x has a value from 3-6 are reported in the US pat 5968976 by AnorMED (Canada) for the treatment of hyperphosphataemia by administration into the gastrointestinal tract. A process for the preparation of lanthanum carbonate tetrahydrate was also disclosed in this patent. Phosphate binding studies in this patent showed that samples of lanthanum carbonate with 3.8 to 4.4 moles of hydration are quicker in phosphate removal.

Chemical abstract search shows that the lanthanum carbonate is reported for the first time in 1923 (Z. Anaorg. Allgem. Chem., 131, 275-86, 1923). In the abstract no information regarding its preparation is disclosed. Crystal structure of lanthanum carbonate is reported in Geol. Nauk, #4, 157-95, 1954. No information regarding the preparation or degree of hydration is mentioned in the relevant abstract of this reference.

A process for the preparation of lanthanum carbonate is given in J. Am. Chem. Soc., 72, 3306, 1950. It is mentioned that pure crystalline rare earth compounds are difficult to prepare by the two commonly used methods, namely the precipitation of the compounds by alkali carbonates or bicarbonates from rare earth salt solutions or the conversion, in aqueous suspension, of rare earth hydroxides to carbonates by carbon dioxide. To solve these problems rare earth trichloroacetates were taken in water medium and heated to get pure carbonates. Here, the by-products are water and chloroform.

2La(C₂Cl₃O₂)₃ + 3H₂O → 3CO₂ + 6CHCl₃ + La₂(CO₃)₃

A process for the preparation of lanthanum carbonate octahydrate is disclosed in Izv. Akad. Nauk. SSSR, Neorgan. Materialy, 1(7), 1166-70 (1965). According to this process lanthanum nitrate is reacted with ammonium carbonate and the product isolated as octahydrate.

A process for the preparation of rare earth carbonates is discussed in J. Inorg. Nucl. Chem., 27, 1489-1493, 1965. According to this process water solutions of rare earth chlorides are reacted with ammonium trichloroacetate (prepared from ammonia and trichloroacetic acid) to get rare earth carbonates. Lanthanum carbonate prepared according to this process was isolated as octahydrate and its IR spectrum, thermogravimetric curve were given. Product was obtained in 50% yield.

Main drawback in this process is the usage of costly trifluoroacetic acid and low yield.

A process for the preparation of lanthanum carbonate is disclosed in US pat 5,968,976. According to this reference, lanthanum oxide is converted to lanthanum nitrate using nitric acid. The resultant aqueous solution was reacted with sodium carbonate to get lanthanum carbonate. Alternatively, lanthanum oxide was reacted with hydrochloric acid to get lanthanum chloride. The resultant aqueous solution was reacted with sodium carbonate to get lanthanum carbonate.

Main drawback in this process is the usage of inorganic base, sodium carbonate. Removal of sodium salts from lanthanum carbonate is difficult. Also, lanthanum carbonate formed in the reaction is slimy in nature and filtration/washing of sodium nitrate is tedious.

Journal of South China University of Technology, 25 (10), 23-26, 1997, describes the precipitation of lanthanum carbonate by addition of a solution of twice the molar equivalent of ammonium bicarbonate to a 0.1 mol/l solution of lanthanum chloride. The mixed solutions are kept at 25 °C for one week, then the precipitate is filtered off, washed with water repeatedly and air-dried. The obtained lanthanum carbonate is the "normal" carbonate form, i.e. having 8 molecules of water per mole of lanthanum carbonate.

Keeping in view of the difficulties in commercialization of the above-mentioned processes for the preparation of lanthanum carbonate, we aimed to develop a simple and cost effective process for commercial production of highly pure lanthanum carbonate.

### SUMMARY OF PRESENT INVENTION

Lanthanum carbonate hydrate is prone to decarboxylation under certain stressful conditions such as high temperature and humidity. The decarboxylation product is lanthanum hydroxycarbonate. Lanthanum hydroxycarbonate is known to exist in two polymorphic forms. An assay method for the quantification of lanthanum hydroxycarbonate in lanthanum carbonate hydrate by powder x-ray analysis is disclosed in EP1852695.

In the preparation of lanthanum carbonate disclosed in US pat 5,968,976, lanthanum carbonate octahydrate is produced by reacting lanthanum chloride or nitrate with sodium carbonate in water medium. The resultant octahydrate is dried carefully at 80°C for various durations of time to get the tetrahydrate derivative of lanthanum carbonate. Under these conditions, formation of lanthanum hydroxycarbonate is unavoidable.

We observed that lanthanum carbonate dihydrate exhibit improved performance over standard lanthanum carbonate tetrahydrate in phosphate binding studies.

One aspect of the present invention is the use of lanthanum carbonate dihydrate for the preparation of medicament for the treatment of hyperphasphataemia by administration into the gastrointestinal tract.

The present invention also provides a pharmaceutical composition comprising lanthanum carbonate dihydrate in admixture or association with a pharmaceutically acceptable diluent or a carrier, in a form suitable for administration into the gastrointestinal tract for the treatment of hyperphosphataemia.

We have now observed that lanthanum carbonate hydrate can be prepared by reacting readily available lanthanum chloride with an organic base such as ammonium bicarbonate to get free-flowing and fine crystalline lanthanum carbonate hydrate. Lanthanum carbonate hydrate can be easily isolated from the reaction mass by simple filtration and washing with minimum amount of water to remove the by-product, ammonium chloride. Also, we have now invented that a selected dihydrate of lanthanum carbonate can be obtained readily by drying under azeotropic conditions using a hydrocarbon solvent. Process for the preparation of dihydrate is robust without requiring any special/controlled drying condition as mentioned in US pat 5,968,976 for similar hydrates.

According to one aspect, the present invention provides a process for the preparation of lanthanum carbonate dihydrate free of lanthanum hydroxycarbonate impurity, which comprises:
(i) Reacting lanthanum chloride hydrate with ammonium bicarbonate at 20-60°C in water medium
(ii) Filtering the resultant lanthanum carbonate hydrate
(iii) Washing the wet lanthanum carbonate hydrate with water to get rid of the chlorides
(iv) Partial drying of lanthanum carbonate hydrate at 60-65°C
(v) Suspending the partially dried lanthanum carbonate hydrate in a hydrocarbon solvent
(vi) Refluxing the medium under azeotropic conditions in the presence/absence of vaccum to get lanthanum carbonate dihydrate
(vii) Filtering and drying of the resultant lanthanum carbonate dihydrate at 60-65°C

The reaction between lanthanum chloride and ammonium bicarbonate is given in the following equation:

2LaCl₃ + 6NH₄(HCO₃) → La₂(CO₃)₃ + 6NH₄Cl + 3CO₂ + 3H₂O

Amount of water used in step (i) is selected from 30-70 volumes to the weight of lanthanum chloride, preferably 50-70 volumes. Preferred temperature of reaction in step (i) is 20-40°C, more preferably 25-35°C. Hydrocarbon solvent used in step (v) and (vi) is selected from hexane, heptane, cyclohexane, toluene, xylene, etc., preferably toluene.
Lanthanum carbonate hydrate produced according to the present invention is free flowing in nature and filtration was very fast compared to lanthanum carbonate hydrate produced according to US pat 5,968,976 process. Also, lanthanum carbonate hydrate produced according to US pat 5,968,976 is slimy in nature. Lanthanum carbonate dihydrate produced according to the present invention is free of lanthanum hydroxycarbonate impurity. Whereas lanthanum carbonate produced according to US pat 5968976 is always contaminated with about 0.5-1.0% of this impurity. Lanthanum carbonate dihydrate is found to be stable at room temperature.

### BRIEF DESCRIPTION OF THE DRAWINGS

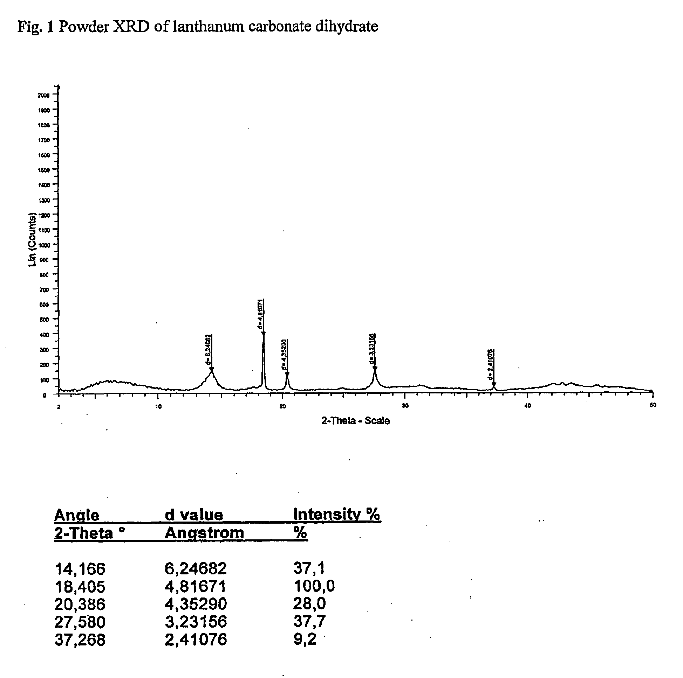

**Fig.1** Powder XRD of lanthanum carbonate dihydrate.

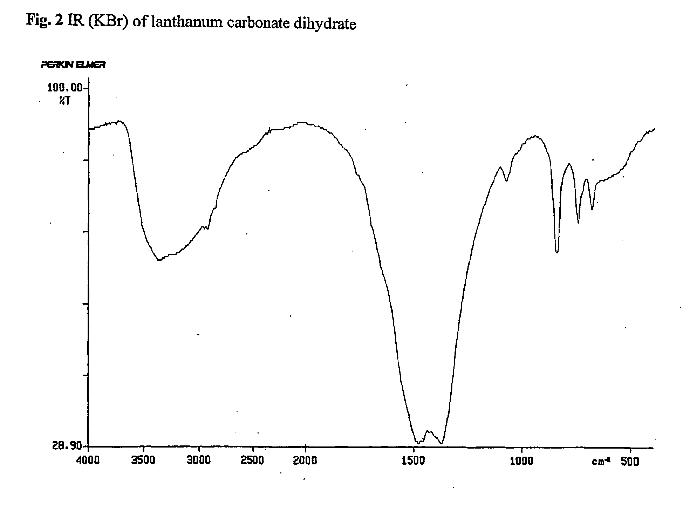

**Fig. 2** IR (KBr) of lanthanum carbonate dihydrate

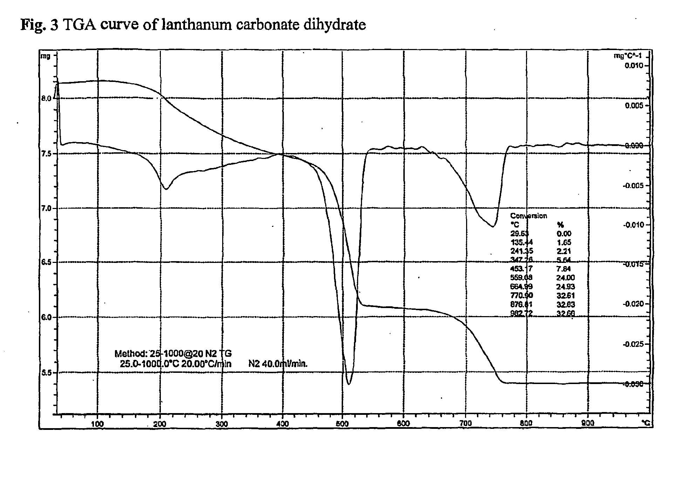

**Fig. 3** TGA curve of lanthanum carbonate dihydrate

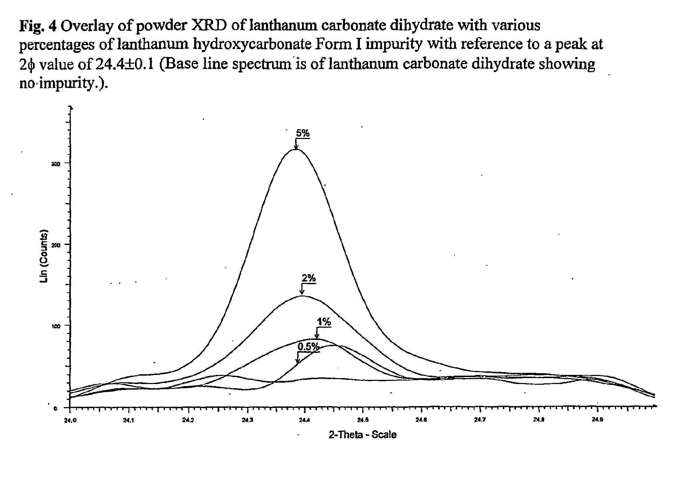

**Fig. 4** Overlay of powder XRD of lanthanum carbonate with various percentages of lanthanum hydroxycarbonate Form I impurity with reference to a peak at 24.4 2 theta value

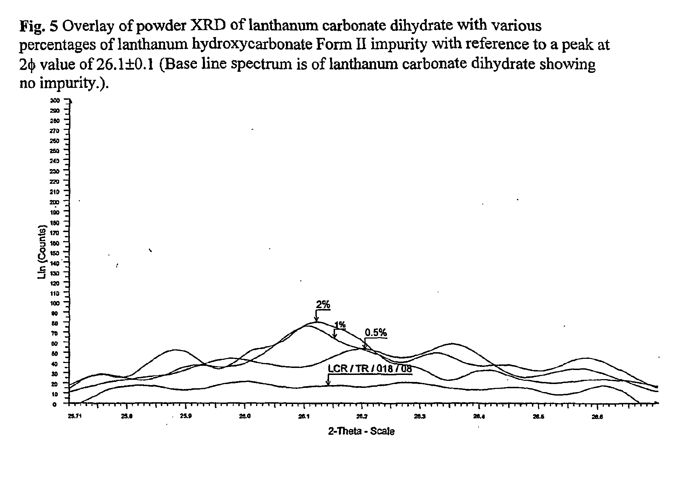

**Fig. 5** Overlay of powder XRD of lanthanum carbonate with various percentages of lanthanum hydroxycarbonate Form II impurity with reference to a peak at 26.1 2-theta value

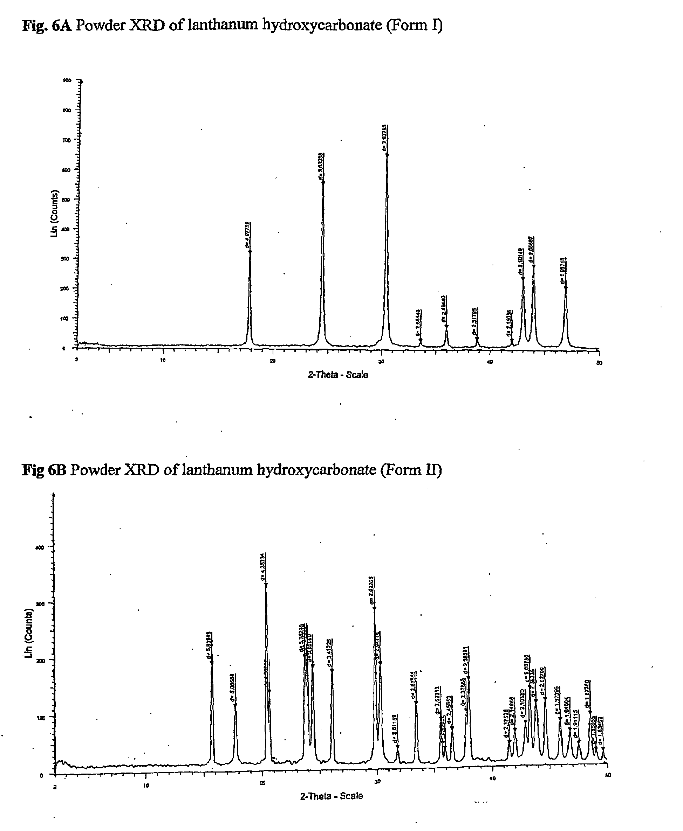

**Fig. 6** Powder XRD of lanthanum hydroxycarbonate (Form I)
**Fig. 7** Phosphate binding capability of lanthanum carbonate hydrates

The details of the invention are given in the Examples given below which are provided to illustrate the invention only and therefore should not be construed to limit the scope of the present invention.

### EXAMPLES

### Example 1

### Preparation of lanthanum carbonate dihydrate

Into a 10L, three-necked RB flask was charged 7L of demineralized water. Lanthanum chloride heptahydrate (100 g) was charged into the flask and stirred for 30min at 25-30°C. The solution was filtered through Bucher funnel and flask under vacuum to get a particle-free solution. Filtrate was transferred into a 10L, three-necked RB flask.

Ammonium bicarbonate (130 g) was charged into a 2L, three-necked RB flask and 700ml of demineralized water was added. The resultant solution was filtered using a funnel and flask to make it particle-free. The filtrate was taken into an addition funnel and added slowly in 3-4 hours into lanthanum chloride solution at 25-30°C. After the completion of addition reaction, the mass was maintained for 1 hour at 25-30°C. The reaction mass was filtered through Buckner funnel and flask under vacuum. The wet cake was washed with 200ml of demineralized water. The wet material was transferred into a 1L, three-necked RB flask, 500 ml of demineralized water was added and stirred for 15 min. The mass was filtered though Buckner funnel and flask under vacuum. The chloride content in wet lanthanum carbonate hydrate was checked. The same washing procedure was repeated one more time, if the chloride content is above 500 ppm. The wet material was transferred into a petridish and dried in an oven at 60-65°C for 4-6 hours.

The above lanthanum carbonate hydrate (69 g) was charged into a 1L, four-necked RB flask. Particle free toluene (400ml) was charged into the flask and the reaction mass was heated to reflux. Water was collected azeotropically using a Dean-Stark apparatus. When the water collection stopped (nearly 3-4 hours) the reaction mass was cooled to 30-35°C. The mass was filtered through Buchner funnel and flask under vacuum and finally washed with 100ml of filtered toluene. The wet material was dried in an oven at 60-65°C for 4-6h to get lanthanum carbonate dihydrate (60 g) as white crystalline solid.

### Example 2

### Preparation of lanthanum carbonate tablets

The lanthanum carbonate tablets were prepared using the above lanthanum carbonate dihydrate.

**Table A**

| Ingredient | 1.000 mg Tablet | 750 mg Tablet | 500 mg Tablet | Function |
|---|---|---|---|---|
| Lanthanum carbonate dihydrate | 1777.50 mg | 1333.12 mg | 888.75 mg | Active ingredient |
| Dextrates (Hydrated) USP/NF | 2102.50 mg | 1576.88 mg | 1051.25 mg | Diluent |
| Talc USP | 30.00 mg | 22.50 mg | 15.00 mg | Glidant |
| Colloidal silicon dioxide USP/NF | 30.00 mg | 22.50 mg | 15.00 mg | Glidant |
| Magnesium stearate USP/NF | 60.00 mg | 45.00 mg | 30.00 mg | Lubricating agent |
| Water | Qs | Qs | Qs | Vehicle |
| Total | 4000 mg | 3000 mg | 2000 mg | |

**Table B**

| Ingredient | 1000 mg Tablet | 750 mg Tablet | 500 mg Tablet | Function |
|---|---|---|---|---|
| Lanthanum carbonate Dihydrate | 1777.50 mg | 1333.13 mg | 888.75 mg | Active ingredient |
| Dextrates (Hydrated) USP/NF | 821.70 mg | 616.27 mg | 410.85 mg | Diluent |
| Dextrates (Hydrated) USP/NF | 105.80 mg | 79.35 mg | 52.90 mg | Binder |
| Talc USP | 30.00 mg | 22.50 mg | 15.00 mg | Glidant |
| Colloidal silicon dioxide USP/NF | 25.00 mg | 18.75 mg | 12.50 mg | Glidant |
| Magnesium stearate USP/NF | 40.00 mg | 30.00 mg | 20.00 mg | Lubricating agent |
| Water | Qs | Qs | Qs | Vehicle |
| Total | 2800 mg | 2100 mg | 1400 mg | |

### Example 3

### Phosphate binding studies of various lanthanum carbonate hydrates

To study the phosphate binding activity of lanthanum carbonate dihdyrate, other hydrates of lanthanum carbonate (monohydrate, tetrahydrate and hexahydrate) were prepared.
a) A stock solution of standard phosphate was prepared by dissolving 13.75 g of Na₂HPO₄ in 1L deionised water after adjusting the pH to 3.0 with conc. HCl.
b) Above stock solution (5 ml) was diluted with 90 ml water adjusted the pH to 3.0 with conc. HCl and made up to 100 ml with water.
c) A two-fold molar excess of Lanthanum carbonate hydrate over phosphate was weighed accurately according to the molecular weight and added to solution b).
d) Sampling was carried out at different time intervals 1, 3, 5, 7, and 10 min. The results are shown in Table C below.

The results show that:
1. Phosphate binding is always relatively very fast with the dihydrate at all points.
2. Peak phosphate binding exceeding 99% is achieved wit the dihydrate in about 7 times whereas only 81 % is achieved with the tetrahydrate.

**Table C: Phosphate binding studies with various hydrates of lanthanum carbonate**

| Time (Minutes) | % Phosphate removed by | | | |
|---|---|---|---|---|
| | Monohydrate | Dihydrate | Tetrahydrate | Hexahydrate |
| 1 | 82.73 | 94.00 | 64.05 | 57.10 |
| 3 | 90.05 | 93.00 | 73.64 | 60.10 |
| 5 | 87.10 | 93.00 | 87.20 | 79.73 |
| 7 | 91.72 | 99.45 | 81.21 | 76.12 |
| 10 | 98.30 | 99.17 | 82.80 | 81.15 |

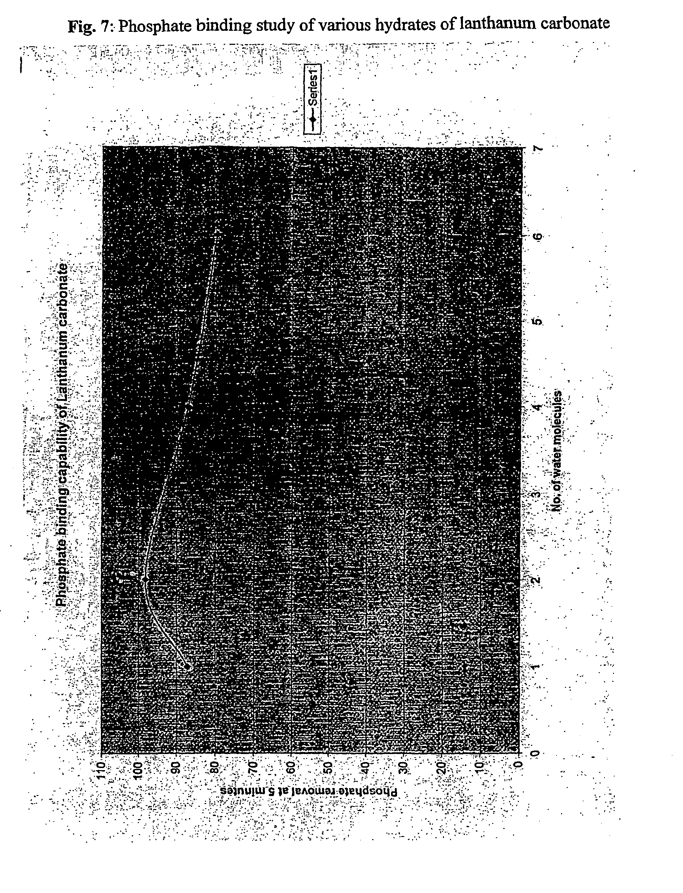

The above results are also plotted and shown in figure 7.

### ADVANTAGES OF PRESENT INVENTION

1. The present invention provides a process for the preparation of lanthanum carbonate dihydrate free of lanthanum hydroxycarbonate impurity.
2. Lanthanum carbonate dihydrate produced according to the process of present invention exhibit improved performance over standard lanthanum carbonate tetrahydrate in phosphate binding studies.
3. Lanthanum carbonate dihydrate is useful for the treatment of hyperphosphataemia in patients with renal failure.

## Claims

1. A process for the preparation of stable lanthanum carbonate of the formula
La₂(CO₃)₃.xH₂O
wherein x = 2.0±0.2
which comprises:
(i) reacting aqueous lanthanum chloride hydrate with aqueous ammonium bicarbonate at 20-60°C
(ii) isolating the resultant lanthanum carbonate hydrate by filtration
(iii) washing the wet lanthanum carbonate hydrate with water to get rid of the chlorides
(iv) partial drying of lanthanum carbonate hydrate at 60-65°C
(v) suspending the partially dried lanthanum carbonate hydrate in a hydrocarbon solvent
(vi) refluxing the medium under azeotropic conditions in the presence/absence of vacuum to get lanthanum carbonate dihydrate
(vii) filtering and drying of the resultant lanthanum carbonate dihydrate at 60-65°C.

2. The process according to claim 1 wherein the amount of water used in step (i) is 30-70 volumes to the weight of lanthanum chloride, preferably 50-70 volumes.

3. The process according to claim 1 or 2 wherein the temperature of reaction in step (i) is 20-40°C, preferably 25-35°C.

4. The process according to any one of claims 1-3 wherein the amount of water present in lanthanum carbonate after partial drying is 15-20% w/w.

5. The process according to any one of claims 1-4 wherein the hydrocarbon solvent used in step (v) and (vi) is selected from hexane, heptane, cyclohexane, toluene and xylene, and preferably is toluene.

6. The process according to any one of claims 1-5 wherein the amount of water present in the lanthanum carbonate dihydrate product is 7.29±0.70% w/w.

7. A stable lanthanum carbonate hydrate of formula La₂(CO₃)₃.xH₂O, x = 2.0±0.2, having a peak phosphate binding capacity of more than 99%.

8. A pharmaceutical composition comprising a lanthanum carbonate as defined in claim 7.

9. A pharmaceutical composition for the treatment of hyperphosphataemia comprising lanthanum carbonate dihydrate of the formula
La₂(CO₃)₃.xH₂O
wherein x = 2.0±0.2.

10. A pharmaceutical composition as defined in claim 9 wherein the lanthanum carbonate dihydrate has a peak phosphate binding capacity of more than 99%.

11. Use of a lanthanum carbonate as defined in any one of claims 7 to 10 in the manufacture of a medicament for use in the treatment of hyperphosphataemia.

## Patentansprüche

1. Verfahren für die Herstellung eines stabilen Lanthancarbonats der Formel
La₂(CO₃)₃.xH₂O
worin x = 2,0±0,2,
das umfasst:
(i) Umsetzen von wässrigem Lanthanchloridhydrat mit wässrigem Ammoniumbicarbonat bei 20-60°C
(ii) Isolieren des resultierenden Lanthancarbonathydrats durch Filtration
(iii) Waschen des nassen Lanthancarbonathydrats mit Wasser, um die Chloride zu beseitigen
(iv) teilweises Trocknen des Lanthancarbonathydrats bei 60-65°C
(v) Suspendieren des teilweise getrockneten Lanthancarbonathydrats in einem Kohlenwasserstofflösungsmittel
(vi) Refluxieren des Mediums unter azeotropen Bedingungen in der Gegenwart/Abwesenheit von Vakuum, um Lanthancarbonatdihydrat zu erhalten
(vii) Abfiltrieren und Trocknen des resultierenden Lanthancarbonatdihydrats bei 60-65°C.

2. Verfahren gemäß Anspruch 1, wobei die in Schritt (i) verwendete Menge an Wasser 30-70 Volumina zu dem Gewicht an Lanthanchlorid, vorzugsweise 50-70 Volumina, ist.

3. Verfahren gemäß Anspruch 1 oder 2, wobei die Reaktionstemperatur in Schritt (i) 20-40°C, vorzugsweise 25-35°C, ist.

4. Verfahren gemäß einem der Ansprüche 1 bis 3, wobei die Menge an Wasser, die nach dem teilweisen Trocknen in Lanthancarbonat vorliegt, 15-20% G/G ist.

5. Verfahren gemäß einem der Ansprüche 1 bis 4, wobei das in Schritt (v) und (vi) verwendete Kohlenwasserstofflösungsmittel aus Hexan, Heptan, Cyclohexan, Toluol und Xylol ausgewählt ist, und vorzugsweise Toluol ist.

6. Verfahren gemäß einem der Ansprüche 1 bis 5, wobei die Menge an Wasser, die in dem Lanthancarbonatdihydratprodukt vorliegt, 7,29±0,70% G/G ist.

7. Stabiles Lanthancarbonathydrat der Formel La₂(CO₃)₃.xH₂O, x = 2,0+0,2, das eine Spitzenphosphatbindekapazität von mehr als 99% hat.

8. Pharmazeutische Zusammensetzung, die ein Lanthancarbonat, wie in Anspruch 7 definiert ist, umfasst.

9. Pharmazeutische Zusammensetzung für die Behandlung von Hyperphosphatämie, die Lanthancarbonatdihydrat der Formel
La₂(CO₃)₃.xH₂O
worin x = 2,0±0,2 ist, umfasst.

10. Pharmazeutische Zusammensetzung, wie in Anspruch 9 definiert ist, wobei das Lanthancarbonatdihydrat eine Spitzenphosphatbindekapazität von mehr als 99% hat.

11. Verwendung eines Lanthancarbonats wie in einem der Ansprüche 7 bis 10 definiert ist, in der Herstellung eines Medikaments für die Verwendung in der Behandlung von Hyperphosphatämie.

## Revendications

1. Procédé de préparation de carbonate de lanthane stable de formule :
La₂(CO₃)₃.xH₂O
dans laquelle x vaut 2,0 ± 0,2,
lequel procédé comporte les étapes suivantes :
i) faire réagir du chlorure de lanthane hydraté, en solution aqueuse, avec du bicarbonate d'ammonium en solution aqueuse, à une température de 20 à 60 °C ;
ii) isoler par filtration le carbonate de lanthane hydraté résultant ;
iii) laver avec de l'eau le carbonate de lanthane hydraté humide, pour le débarrasser des ions chlorure ;
iv) sécher partiellement le carbonate de lanthane hydraté, à une température de 60 à 65 °C ;
v) mettre ce carbonate de lanthane hydraté, partiellement séché, en suspension dans un solvant de type hydrocarbure ;
vi) porter le mélange au reflux dans des conditions azéotropiques, sous vide ou non, pour obtenir du carbonate de lanthane dihydraté ;
vii) et séparer par filtration le carbonate de lanthane dihydraté résultant et le faire sécher à une température de 60 à 65 °C.

2. Procédé conforme à la revendication 1, dans lequel la quantité d'eau utilisée dans l'étape (i), par rapport au poids du chlorure de lanthane, vaut de 30 à 70 volumes, et de préférence, de 50 à 70 volumes.

3. Procédé conforme à la revendication 1 ou 2, dans lequel, dans l'étape (i), la température de réaction vaut de 20 à 40 °C, et de préférence, de 25 à 35 °C.

4. Procédé conforme à l'une des revendications 1 à 3, dans lequel la proportion d'eau présente dans le carbonate de lanthane après le séchage partiel vaut de 15 à 20 % en poids/poids.

5. Procédé conforme à l'une des revendications 1 à 4, dans lequel le solvant de type hydrocarbure utilisé dans les étapes (v) et (vi) est choisi parmi les hexane, heptane, cyclohexane, toluène et xylène, la préférence allant au toluène.

6. Procédé conforme à l'une des revendications 1 à 5, dans lequel la proportion d'eau présente dans le carbonate de lanthane dihydraté obtenu comme produit vaut 7,29 ± 0,70 % en poids/poids.

7. Carbonate de lanthane hydraté stable, de formule
La₂(CO₃)₃.xH₂O
dans laquelle x vaut 2,0 ± 0,2,
qui présente un pouvoir maximal de fixation des phosphates de plus de 99 %.

8. Composition pharmaceutique comprenant un carbonate de lanthane conforme à la revendication 7.

9. Composition pharmaceutique conçue pour le traitement d'une hyperphosphatémie, comprenant du carbonate de lanthane dihydraté de formule
La₂(CO₃)₃.xH₂O
dans laquelle x vaut 2,0 ± 0,2.

10. Composition pharmaceutique conforme à la revendication 9, dans laquelle le carbonate de lanthane dihydraté présente un pouvoir maximal de fixation des phosphates de plus de 99 %.

11. Utilisation d'un carbonate de lanthane tel que défini dans l'une des revendications 7 à 10 dans la fabrication d'un médicament conçu pour être utilisé dans le traitement d'une hyperphosphatémie.
